# EUROPÄISCHE PATENTSCHRIFT

(11) **EP 0 807 619 B1**
(45) Veröffentlichungstag und Bekanntmachung des Hinweises auf die Patenterteilung: **11.09.2002**
(21) Anmeldenummer: 97107407.5
(22) Anmeldetag: 12.05.1997
(51) Int. Cl.: C07C 68/00, C07C 69/96

(54) **Verfahren zur Herstellung von Diarylcarbonaten**
Process for the production of diaryl carbonates
Procédé de fabrication de carbonates de diaryle

(30) Priorität: 17.05.1996 DE 19619949
(43) Veröffentlichungstag der Anmeldung: 19.11.1997
(73) Patentinhaber: BAYER AG, 51368 Leverkusen (DE)
(72) Erfinder: Buysch, Hans-Josef, Dr., 47809 Krefeld (DE); Hesse, Carsten, Dr., 47800 Krefeld (DE); Rechner, Johann, Dr., 47906 Kempen (DE)

(56) Entgegenhaltungen:
- EP-A- 0 583 938
- DE-A- 2 815 512

## Beschreibung

Die vorliegende Erfindung betrifft ein Verfahren zur Herstellung von Diarylcarbonaten durch Umsetzung einer aromatischen Hydroxyverbindung (z.B. Phenol) mit Kohlenmonoxid und Sauerstoff in Gegenwart eines Katalysators, eines Cokatalysators, eines quaternären Salzes und einer Base, das dadurch gekennzeichnet ist, dass man die Reaktion in einer Schmelze aus dem Diarylcarbonat und der dem Carbonat zugrundeliegenden Hydroxyverbindung durchführt und dieser Reaktionsmischung vor der Aufarbeitung gegebenenfalls noch weiteres Diarylcarbonat zusetzt.

Es ist bekannt, organische Carbonate durch oxidative Umsetzung einer aromatischen Hydroxyverbindung mit Kohlenmonoxid in Gegenwart eines Edelmetall-Katalysators herzustellen (DE-A 27 38 437). Als Edelmetall wird bevorzugt Palladium eingesetzt. Zusätzlich können ein Cokatalysator (z.B. Mangan- oder Kobaltsalze), eine Base, ein quaternäres Salz, verschiedene Chinone bzw. Hydrochinone und Trockenmittel eingesetzt werden. Dabei kann in einem Lösungsmittel gearbeitet werden. Bevorzugt wird hierfür Methylenchlorid verwendet.

Die mit diesem Verfahren erzielten Raum-Zeit-Ausbeuten sind sehr gering und für eine technische Anwendung nicht akzeptabel. Weiterhin erschweren die hohe Verdünnung, die Verwendung von Molsieb und die große Katalysatormenge die Aufarbeitung in erheblichem Maße und machen das Verfahren zusätzlich unwirtschaftlich.

Höhere Diarylcarbonatgehalte in den Reaktionslösungen werden, falls überhaupt, erst nach mehrstündigen Reaktionszeiten erreicht (JP-01 165 551, WO 93/03000, EP-A 583 935, EP-A 583 937 und EP-A 583 938), obwohl bei hohen Temperaturen und Drücken gearbeitet wird. Lange Reaktionszeiten führen zu großen Reaktionsvolumina und machen die Verfahren unattraktiv. Zur Wasserabtrennung wird weiterhin der Zusatz von Molsieb vorgeschlagen, um die Zersetzung des gebildeten Diarylcarbonats und eine Deasaktivierung des Katalysators zu vermeiden. Die Verwendung von Molsieb macht eine technische Nutzung der Verfahren unattraktiv, da für eine effektive Abtrennung des Wassers aus der Flüssigphase große Mengen Molsieb (100 - 500 % Überschuss, bezogen auf die Trocknungskapazität und die erwartete Wassermenge) benötigt werden, die unter hohem technischen Aufwand regeneriert werden müssen. In Anwesenheit genügender Mengen Molsieb, wie sie in den genannten Anmeldungen eingesetzt werden, lässt sich der Wassergehalt in einer Reaktionslösung auf wenige ppm beschränken. Da trotzdem Diarylcarbonat-Zersetzung beobachtet wird, liegt es nahe, dass selbst Wasserspuren zu einer raschen Diarylcarbonatzersetzung führen. Konsequenterweise vermeidet man daher zur Unterdrückung der Hydrolysereaktion auch gleichzeitig hohe Konzentrationen an Diarylcarbonat in der Reaktionsmischung, zumal sich aus EP-A 583 935 entnehmen lässt, dass mit zunehmender Diphenylcarbonatkonzentration in der Reaktionsmischung die Reaktion erheblicher langsamer abläuft.

In JP-04 257 546 wird ein Verfahren beschrieben, bei dem man organische Carbonate durch oxidative Umsetzung einer aromatischen Hydroxyverbindung mit Kohlenmonoxid in Gegenwart eines Edelmetall-Katalysators und eines quaternären Salzes durch kontinuierliches Einspeisen in eine Destillationskolonne bei 150-205°C und 30-50 bar erhält. Das Reaktionswasser destilliert hierbei kontinuierlich ab.

Nachteilig bei diesem Verfahren ist, dass man zur Entfernung des Reaktionswassers in einer Destillationskolonne arbeiten muss, die aufgrund ihrer Konstruktion nur kurze Verweilzeiten ermöglicht. Die mit diesem Verfahren realisierbaren Raum-Zeit-Ausbeuten sind dem zufolge mit nur 17,8 g/lh sehr niedrig. Mit der Reaktionsführung in einer Destillationskolonne ist die Verwendung großer Mengen Halogenide bei hohen Temperaturen (150 - 205°C) verbunden. Dies führt zu großen Korrosionsproblemen, die zusätzlich einen hohen apparativen Aufwand bedingen. Dem Fachmann ist außerdem bekannt, dass unter den angegebenen Reaktionsbedingungen das bevorzugt eingesetzte Jodid nicht stabil ist und in erheblichem Maße zu Jod oxidiert wird. Dies führt zu großen Verlusten des quaternären Salzes und zur Bildung von Nebenprodukten, was die Selektivität und damit die Wirtschaftlichkeit dieses Verfahrens stark beeinträchtigt. Bei den benötigten hohen Temperaturen und Drücken ist außerdem mit einer raschen Desaktivierung des homogenen Katalysatorsystems, verursacht durch die Halogenverluste und das Teilchenwachstum des Palladiums zu rechnen, so dass eine wirtschaftliche Nutzung dieses Verfahrens nicht möglich ist. Weiterhin liefert auch dieses Verfahren nur sehr geringe Diarylcarbonatkonzentrationen und besitzt somit keinen Vorteil bei der Diarylcarbonatisolierung gegenüber den bereits diskutierten Verfahren. Zur Verminderung der Hydrolyseverluste vermeidet man offensichtlich auch bei diesem Verfahren hohe Diarylcarbonatgehalte.

In EP-A 667 336 wird ein Verfahren zur Wasserentfernung durch Strippen mit überschüssigem Reaktionsgas bei 40-120°C und 2-50 bar beschrieben.

Nachteilig auch bei diesem Verfahren ist, dass Diarylcarbonatgehalte über 20 Gew.-% erst nach mehrstündigen Reaktionszeiten erreichbar sind. Weiterhin ist dieses Verfahren offensichtlich für hohe Diarylcarbonatkonzentrationen nicht geeignet, da die Wasserentfernung durch Strippen mit überschüssigem Reaktionsgas nicht die Trocknungswirkung der Molsiebe besitzt und hierdurch eine wesentlich stärkere Hydrolysereaktion zu erwarten ist.

Die Isolierung des gebildeten Diarylcarbonats aus den Reaktionsmischungen erfordert bei geringen Gehalten an Diarylcarbonat einen erheblichen Energieaufwand, da große Mengen Lösungsmittel bzw. aromatische Hydroxyverbindung abgetrennt werden müssen.

Es bestand daher Bedarf für ein Verfahren, das es erlaubt, innerhalb kurzer Reaktionszeiten hohe Diarylcarbonatgehalte in der Reaktionsmischung zu erzielen, um die nachgeschaltete Isolierung des Diarylcarbonats mit minimalem Energieaufwand durchführen zu können.

Es wurde nun gefunden, dass die beschriebenen Nachteile des Standes der Technik überwunden werden können, wenn man die Reaktion in einer Schmelze aus Diarylcarbonat und der entsprechenden Hydroxyverbindung durchführt und hierdurch zu Beginn der Reaktion bereits hohe Diarylcarbonatgehalte im Reaktionssystem vorliegen. Die Reaktion läuft auch bei hohen Gehalten an Diarylcarbonat in der Schmelze noch mit hohen Raum-Zeit-Ausbeuten ab. Entgegen der Erwartung treten auch bei hohen Diarylcarbonatgehalten kaum Hydrolyse- oder Nebenreaktionen auf. Zur Vermeidung der Hydrolyse des Diarylcarbonats genügt beispielsweise eine Entfernung des bei der Reaktion gebildeten Wassers durch Strippen mit überschüssigem Reaktionsgas. Für die anschließende Aufarbeitung kann die Zugabe weiteren Diarylcarbonats nach Abschluss der Reaktion von Vorteil sein.

Die Erfindung betrifft demnach ein Verfahren zur Herstellung eines organischen Carbonats der Formel

R-O-CO-O-R (I)

in der
- R: ein substituiertes oder nicht substituiertes C₆-C₁₂-Aryl, bevorzugt ein substituiertes oder nicht substituiertes Phenyl, besonders bevorzugt nicht substituiertes Phenyl bedeutet,
durch Umsetzung einer aromatischen Hydroxyverbindung der Formel

R-O-H (II)

worin
- R: die oben angegebene Bedeutung hat,
mit Kohlenmonoxid und Sauerstoff in Gegenwart eines Platinmetall-Katalysators, eines Cokatalysators, eines quaternären Salzes und einer Base bei einer Temperatur von 30 bis 200°C, bevorzugt 30 bis 150°C, besonders bevorzugt 40 bis 120°C und einem Druck von 1 bis 200 bar, bevorzugt 2 bis 150 bar, besonders bevorzugt 5 bis 75 bar, das dadurch gekennzeichnet ist, dass man die Reaktion in einer Schmelze aus Diarylcarbonat und aromatischer Hydroxyverbindung durchführt, die schon zu Beginn der Reaktion einen Gehalt an Diarylcarbonat von mindestens 20 Gew.-%, bevorzugt mindestens 30 Gew.-% und besonders bevorzugt mindestens 40 Gew.-% aufweist, und aus der Schmelze nach im Prinzip bekannten Verfahren Diarylcarbonat isoliert.

Im erfindungsgemäßen Verfahren wird bevorzugt eine Schmelze aus Diarylcarbonat und aromatischer Hydroxyverbindung eingesetzt, die 20 bis 95 Gew.-% Diarylcarbonat, bevorzugt 30 bis 75 Gew.-% Diarylcarbonat, besonders bevorzugt 40 bis 60 Gew.-% Diarylcarbonat enthält.

Diarylcarbonat kann hierbei dem Eduktstrom des Synthesereaktors frisch zugesetzt werden oder als Rückstrom aus der Diarylcarbonatisolierung bereits im Recyclat enthalten sein. Im Falle des Rückstroms aus der Diarylcarbonatisolierung muss dann nach Ergänzung der aromatischen Hydroxyverbindung gegebenenfalls noch durch Zugabe von Diarylcarbonat der gewünschte Gehalt eingestellt werden. Im erfindungsgemäßen Verfahren ist es bei der Diarylcarbonatisolierung nicht notwendig, das gesamte Diarylcarbonat aus der Reaktionsmischung zu entfernen, es genügt die Entnahme des durch die Reaktion entstandenen Diarylcarbonats.

In einer weiteren Ausführungsform kann es vorteilhaft sein, einen Teil des Diarylcarbonats erst unmittelbar vor der Diarylcarbonatisolierung, zuzusetzen. Es wurde nun gefunden, dass es vorteilhaft ist, wenn die Schmelze vor dem Aufarbeitungsschritt mindestens 40 Gew.-%, bevorzugt mindestens 45 Gew.-%, besonders bevorzugt mehr als 50 Gew.-% an Diarylcarbonat enthält. Auch bei Reaktionsmischungen, die nach anderen, im Prinzip bekannten Verfahren hergestellt wurden, wird durch Zugabe von Diarylcarbonat zur Schmelze die Aufarbeitung vorteilhaft beeinflusst.

Die Entfernung des während der Reaktion gebildeten Wassers kann im erfindungsgemäßen Verfahren durch Molsieb, durch destillative Entfernung aus einem Kreisstrom oder durch Strippen mit überschüssigem Reaktionsgas erfolgen. Bevorzugt wird das Wasser durch Strippen mit überschüssigem Reaktionsgas entfernt.

Bei destillativer Entfernung wird ein Teilstrom der Reaktionsmischung entnommen, entspannt und dann weitgehend isotherm bei reduziertem Druck durch Verdampfen des Wassers entwässert und anschließend dem Reaktor wieder zugeführt. Der pro Stunde entnommene Teilstrom an Reaktionsmischung kann im erfindungsgemäßen Verfahren das 0,01 bis 30fache, bevorzugt das 0,05 bis 20fache, besonders bevorzugt das 0,1 bis 10fache des Reaktorinhalts betragen.

In einer weiteren Ausführungsform lässt sich das Wasser durch spontane Verdampfung bei der Entspannung auf reduzierten Druck entfernen. Hierbei kühlt sich der entnommene Teilstrom ab und muss vor der Rückführung wieder auf Reaktionstemperatur erwärmt werden.

In einer weiteren Ausführungsform kann wie in JP-04 257 546 beschrieben in einer Destillationskolonne zur Wasserentfernung gearbeitet werden.

Bevorzugt erfolgt die Entfernung des bei der Reaktion gebildeten Wassers durch Strippen mit überschüssigem Reaktionsgas. Die eingesetzte Menge an Reaktionsgas beträgt hierbei 1 bis 100 000 Nl pro Liter Reaktionsmischung, bevorzugt 5 bis 50 000 Nl pro Liter Reaktionsmischung, besonders bevorzugt 10 bis 10 000 Nl pro Liter Reaktionsmischung.

Das Reaktionsgas besteht im erfindungsgemäßen Verfahren aus Kohlenmonoxid, Sauerstoff und gegebenenfalls einem Inertgas.

Der Anteil an Kohlenmonoxid und Sauerstoff im Reaktionsgas kann in weiten Konzentrationsgrenzen variiert werden, es wird jedoch zweckmäßigerweise ein molares Verhältnis CO:O₂ (normiert auf CO) von 1:0,001 bis 1:0,5, bevorzugt 1:0,01 bis 1:0,4 und besonders bevorzugt von 1:0,02 bis 1:0,3. eingestellt. Der Sauerstoffpartialdruck ist bei diesen Molverhältnissen groß genug, um hohe Raum-Zeit-Ausbeuten erreichen zu können. Die Reaktionsgase unterliegen keinen besonderen Reinheitsanforderungen. So kann Synthesegas als CO-Quelle und Luft als O₂-Träger dienen, es ist jedoch darauf zu achten, dass keine Katalysatorgifte wie Schwefel oder dessen Verbindungen eingetragen werden. In einer bevorzugten Ausführungsform des erfindungsgemäßen Verfahrens werden reines CO und reiner Sauerstoff verwendet.

Inerte Bestandteile des Reaktionsgases im erfindungsgemäßen Verfahren können Stickstoff, Wasserstoff, Kohlendioxid, Edelgase sowie unter Reaktionsbedingungen beständige organische Verbindungen, die gegebenenfalls mit Wasser ein Azeotrop bilden, sein. Die Konzentration an Inertgas im Reaktionsgas beträgt 0 bis 60 Vol.-%, bevorzugt 0 bis 20 Vol.-%, besonders bevorzugt 0 bis 5 Vol.-%.

Beispiele für erfindungsgemäß umsetzbare aromatische Hydroxyverbindungen sind Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt ist Phenol. Erfindungsgemäß einsetzbare aromatische Hydroxyverbindungen können ein- oder zweifach mit C₁-C₄-Alkyl, C₁-C₄-Alkoxy, Fluor, Chlor oder Brom substituiert sein.

Im erfindungsgemäßen Verfahren können sowohl organische als auch anorganische Basen oder Mischungen derselben verwendet werden. Beispiele für anorganische Basen sind Alkalimetallhydroxide und -carbonate, -carboxylate oder andere Salze schwacher Säuren sowie Alkalisalze von aromatischen Hydroxyverbindungen der Formel (II), z.B. Alkalimetallphenolate. Im erfindungsgemäßen Verfahren können auch Hydrate von Alkalimetallphenolaten eingesetzt werden. Als Beispiel für ein solches Hydrat sei Natriumphenolattrihydrat genannt. Die Menge an zugesetztem Wasser ist jedoch vorzugsweise so bemessen, dass pro Mol Base maximal 5 Mol Wasser eingesetzt werden. Höhere Wasserkonzentrationen führen im allgemeinen zu schlechteren Umsätzen und zur Zersetzung der gebildeten Carbonate. Beispiele für organische Basen sind tertiäre Amine, die als organische Reste C₆- bis C₁₀-Aryl-, C₇- bis C₁₂-Aralkyl- und/oder C₁- bis C₂₀-Alkyl-Reste tragen können, Pyridinbasen und hydrierte Pyridinbasen. Beispielhaft seien genannt: Triethylamin, Tripropylamin, Tributylamin, Trioctylamin, Benzyldimethylamin, Dioctylbenzylamin, Dimethylphenethylamin, 1-Dimethylamino-2-phenylpropan, Pyridin, N-Methylpiperidin, 1,2,2,6,6-Pentamethylpiperidin, oder Stickstoffbasen wie Amidine, z.B. DBU (1,8-Diazabicyclo[5.4.0]undec-7-en) und DBN (1,5-Diazabicyclo[4.3.0]non-5-en) oder Guanidine wie TBD (1,5,7-Triazabicyclo[4.4.0]dec-5-en). Bevorzugt wird als Base ein Alkalisalz einer aromatischen Hydroxyverbindung verwendet, besonders bevorzugt ein Alkalisalz der aromatischen Hydroxyverbindung, die auch zum organischen Carbonat umgesetzt werden soll. Diese Alkalisalze können Lithium-, Natrium-, Kalium-, Rubidium- oder Cäsiumsalze sein. Bevorzugt sind Lithium-, Natrium-, und Kaliumphenolat, besonders bevorzugt Natriumphenolat.

Die eingesetzte Menge an Base beträgt 0,01 bis 20 Gew.-%, bezogen auf das Gewicht des Reaktionsgemisches. Vorzugsweise beträgt diese Menge 0,05 bis 15 Gew.-%, besonders bevorzugt 0,1 bis 5 Gew.-%.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol, als auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt. Diese Lösungsmittel können allein oder in beliebiger Kombination miteinander eingesetzt werden. So besteht eine Ausführungsform des erfindungsgemäßen Verfahrensdarin, dass man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatischen Hydroxyverbindung, die zum organischen Carbonat umgesetzt werden soll. Ganz besonders bevorzugt wird die Base in Phenol gelöst zugesetzt.

Das Verhältnis von Base zu Platinmetall, z.B. Palladium, wird vorzugsweise so gewählt, dass pro Mol Platinmetall 0,1 bis 500, bevorzugt 0,3 bis 200, besonders bevorzugt 0,9 bis 130 Äquivalente Base eingesetzt werden.

Das erfindungsgemäße Verfahren wird vorzugsweise ohne Lösungsmittel durchgeführt. Es können aber auch inerte Lösungsmittel verwendet werden. Als Beispiele seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt.

Die für das erfindungsgemäße Verfahren geeigneten Platinmetall-Katalysatoren bestehen aus mindestens einem Metall der Gruppe VIII, vorzugsweise Palladium. Es kann bei dem erfindungsgemäßen Verfahren in verschiedener Form zugegeben werden. Palladium kann in metallischer Form oder in Form von Palladium-Verbindungen der Oxidationsstufen 0 und +2 eingesetzt werden, beispielsweise als Palladium(II)-acetylacetonat, Halogenid, Carboxylat von C₂-C₆-Carbonsäuren, Nitrat, Oxid oder in Form von Komplexverbindungen, die beispielsweise Kohlenmonoxid, Olefine, Amine, Phosphine und Halogenide enthalten können. Besonders bevorzugt sind Palladiumbromid und Palladiumacetylacetonat.

Die eingesetzte Menge an Platinmetall-Katalysator ist im erfindungsgemäßen Verfahren nicht beschränkt. Bevorzugt wird so viel Katalysator zugesetzt, dass die Konzentration des Metalls im Reaktionsansatz 1 - 3000 ppm, besonders bevorzugt 5 - 500 ppm beträgt.

Als Cokatalysator für das erfindungsgemäße Verfahren wird ein Metall der Gruppen III B, IV B, V B, VI B, VII B, VIII B, I B, II B (CAS-Nomenklatur) eingesetzt, wobei das Metall in verschiedenen Oxidationsstufen eingesetzt werden kann. Als Beispiele seien Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von C₂-C₆-Carbonsäuren, Diketonate oder Nitrate eingesetzt werden, oder als Komplexverbindungen, die beispielsweise Kohlenmonoxid, Olefine, Amine, Phosphine und Halogenide enthalten können. Bevorzugt werden Manganverbindungen im erfindungsgemäßen Verfahren verwendet, besonders bevorzugt Komplexe des Mangan(II) und Mangan(III), ganz besonders bevorzugt Mangan(II)acetylacetonat und Mangan(III)acetylacetonat.

Der Cokatalysator wird in einer Menge zugesetzt, dass sein Anteil am Reaktionsgemisch 0,0001 bis 20 Gew.-%, bevorzugt 0,005 bis 5 Gew.-%, besonders bevorzugt 0,01 bis 2 Gew.-% beträgt.

Beispiele für erfindungsgemäß eingesetzte quaternäre Salze sind mit organischen Resten substituierte Ammonium-, Phosphonium- oder Sulfoniumsalze. Geeignet sind Ammonium-, Phosphonium- und Sulfoniumsalze, die als organische Reste C₆- bis C₁₀-Aryl-, C₇- bis C₁₂-Aralkyl- und/oder C₁- bis C₂₀-Alkyl-Reste und als Anion ein Halogenid, Tetrafluoroborat oder Hexafluorophosphat aufweisen. Bevorzugt werden Ammoniumsalze verwendet, die als organische Reste C₆- bis C₁₀-Aryl-, C₇bis C₁₂-Aralkyl- und/oder C₁- bis C₂₀-Alkyl-Reste und als Anion ein Halogenid aufweisen, besonders bevorzugt ist Tetrabutylammoniumbromid. Die Menge eines solchen quaternären Salzes beträgt 0,1 - 20 Gew.-%, des Reaktionsgemisches, bevorzugt 0,5 - 15 Gew.-%, besonders bevorzugt 1 - 5 Gew.-%.

Vor dem Einsatz im erfindungsgemäßen Verfahren wird der Platinmetall-Katalysator aktiviert. Dazu wird die Platinmetall-Verbindung, deren Menge im erfindungsgemäßen Verfahren nicht beschränkt ist, bevorzugt aber so bemessen ist, dass die Konzentration des Platinmetalls im Aktivierungsansatz 0,0001 bis 30 Gew.-%, besonders bevorzugt 0,001 bis 10 Gew.-% beträgt, in einem inerten Lösungsmittel oder direkt in der Schmelze der aromatischen Hydroxyverbindung oder Mischungen derselben gelöst. Zu dieser Lösung wird ein quaternäres Salz aus der Gruppe der oben beschriebenen Verbindungen gegeben. Diese Lösung wird anschließend bei 15 bis 200°C, bevorzugt bei 20 bis 150°C, besonders bevorzugt bei 40 bis 100°C mit Kohlenmonoxid behandelt. Das kann sowohl dadurch geschehen, dass man bei Normaldruck pro Gramm des eingesetzten Platinmetalls Kohlenmonoxid in einer Menge von 0,1 bis 250 l/h, bevorzugt 0,5 bis 200 l/h, besonders bevorzugt 1 bis 100 l/h einleitet, als auch dadurch, dass man die Lösung in einem Autoklaven unter einem Druck von 1 bis 300 bar, bevorzugt 1 bis 200 bar, besonders bevorzugt 1 bis 150 bar mit Kohlenmonoxid versetzt. Die Aktivierungszeit hängt vom verwendeten Platinmetall-Katalysator und einem gegebenenfalls eingesetzten inerten Lösungsmittel ab. Sie beträgt im allgemeinen wenige Minuten bis einige Stunden. Der Platinmetall-Katalysator kann direkt vor der Reaktion aktiviert, aber auch nach Abtrennung des Lösungsmittels oder der aromatischen Hydroxyverbindung, z.B. durch Abdestillieren, isoliert und gelagert werden.

In einer weiteren bevorzugten Ausführungsform werden statt des homogenen Katalysatorsystems heterogene Katalysatoren, bei denen das Platinmetall und gegebenenfalls auch der Cokatalysator auf einem Träger aufgebracht sind, als Pulver oder Formkörper eingesetzt. Die übrigen Komponenten des Katalysatorsystems sind in der Reaktionsmischung homogen gelöst. Die Menge des Platinmetalls am Gesamtgewicht des heterogenen Katalysators beträgt 0,01 bis 15 Gew.-%, bevorzugt 0,05 bis 10 Gew.-%, gerechnet als Platinmetall.

Als Cokatalysatoren auf dem Katalysatorträger wird mindestens eine Metallverbindung der Gruppen IIIB, IVB, VB, VIB, VIIB, VIIIB, IB, IIB des Periodensystems der Elemente (CAS-Notation) oder der Seltenerdmetalle (Atomnummern 58-71) eingesetzt, bevorzugt Mn, Cu, Co, V, Zn, Ce und Mo, besonders bevorzugt werden Mn, Co, Cu, Mo und Ce eingesetzt.

Die Menge der Cokatalysator enthaltenden Verbindung im reaktionsbereiten Zustand beträgt 0,01 bis 15 Gew.-%, bevorzugt 0,05 bis 10 Gew.-%, gerechnet als Metall und bezogen auf das Gesamtgewicht des heterogenen Katalysators.

Als Katalysatorträger eignen sich ein oder mehrere Metalloxide aus der Gruppe von V, Mn, Ti, Cu, Zr, La, der Seltenerdmetalle (Atomnummern 58-71), sowohl im Sinne chemisch einheitlicher Reinsubstanzen als auch im Gemisch, sowie Eisen- und Kobaltoxide, Nickel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvem als Suspension in Rührgefäßen oder Blasensäulen werden Mengen von 0,001 bis 50 Gew.-%, bevorzugt 0,01 bis 20 Gew.-%, besonders bevorzugt 0,1 bis 10 Gew.-% Trägerkatalysator-Pulver, auf die eingesetzte Menge an Reaktionsmischung bezogen, verwendet.

In besonders bevorzugten Ausführungsformen wird der heterogene Trägerkatalysator ortsfest in Rührbehältern, Blasensäulen, einem Rieselphasenreaktor oder Kaskaden dieser Reaktoren eingesetzt. Eine Abtrennung des Trägerkatalysators entfällt dann völlig.

Als Reaktoren für das erfindungsgemäße Verfahren mit homogenem oder heterogenem Katalysator sind Rührkessel, Autoklaven, Destillationskolonnen, Blasensäulen und im Falle von ortsfest eingesetzten heterogenen Katalysatoren auch zusätzlich noch Rieselphasenreaktoren geeignet, wobei diese als Einzelreaktoren oder als Kaskade eingesetzt werden können.

In einer Kaskade können 2 bis 15, bevorzugt 2 bis 10, besonders bevorzugt 2 bis 5 Reaktoren hintereinandergeschaltet sein.

Die Zuführung des Rektionsgases kann im Gegen- oder Gleichstrom zur Flüssigkeitsströmung im Einzelreaktor und in den Kaskadenreaktoren erfolgen. CO- und Sauerstoff können gemeinsam oder getrennt voneinander zudosiert werden, wobei Gasmenge und Zusammensetzung für die einzelnen Reaktoren einer Kaskade auch unterschiedlich sein kann. Es kann gegebenenfalls von Vorteil sein, die Einspeisungsorte von CO, Sauerstoff und gegebenenfalls Inertgas in einem einzelnen Reaktor oder bei den Reaktoren einer Kaskade räumlich zu trennen.

Zur Vermischung der Reaktionskomponenten in Rührbehältern sind diese mit dafür brauchbaren Rührern ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc.

In dem erfindungsgemäßen Verfahren können folgende Typen von Blasensäulen eingesetzt werden: einfache Blasensäulen, Blasensäulen mit Einbauten, z.B. Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (Chem. Ing. Tech. 61 (1979) 208; W.-D. Deckwer: Reaktionstechnik in Blasensäulen, Otto Salle Verlag 1985).

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren.

Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz.

Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer, im Falle eines Rührkessels oder andere bekannte Gasverteilungsorgane in die Reaktionsmischung geleitet. Nach Erreichen des optimalen Umsatzes wird das Reaktionsgemisch aus dem Reaktor entfernt oder gegebenenfalls im Reaktor aufgearbeitet.

Die pulverförmigen Trägerkatalysatoren können aus der Reaktionsmischung z.B. durch Filtration, Sedimentation oder Zentrifugieren abgetrennt werden.

Die in diskontinuierlichen Versuchen verwendeten Trägerkatalysatoren können bei gleichen Einsatzstoffen gegebenenfalls ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugten Ausführungsformen des erfindungsgemäßen Verfahrens kommt eine kontinuierliche Arbeitsweise im Einzelreaktor oder in einer Kaskade aus Reaktoren zum Einsatz. Bei Verwendung ortsfester heterogener Katalysatoren können diese über lange Zeit im Reaktor verbleiben und dort auch gegebenenfalls regeneriert werden. Bei dieser Variante des erfindungsgemäßen Verfahrens entnimmt man kontinuierlich einen den Feedströmen entsprechenden Teil der Reaktionsmischung, wobei der Füllstand im Reaktor konstant gehalten wird, isoliert daraus das im Reaktor gebildete Diarylcarbonat in im Prinzip bekannter Weise, z.B. durch fraktionierte Schmelzkristallisation (EP-A 687 666) oder ein Extraktionsverfahren, und führt die an Diarylcarbonat verarmte Mischung anschließend wieder in den Prozess zurück. Gegebenenfalls wird der entnommene Teil der Reaktionsmischung vor der Isolierung des Diarylcarbonats noch zusätzlich mit Diarylcarbonat versetzt, so dass in der Mischung mindestens 40 Gew.-% Diarylcarbonat enthalten sind. Dadurch wird die Aufarbeitung erleichtert.

### Beispiele

### Beispiel 1: (21,4 % DPC-Zusatz)

In einem Autoklaven (11) mit Begasungsrührer, Wellenbrecher, kontinuierlicher Gasdosierung, Druckhaltung, Kühler und nachgeschalteten Kühlfallen, wurden 0,12 g Palladiumbromid und 11,64 g Tetrabutylammoniumbromid bei 60°C in 150 g DPC und 500 g Phenol gelöst. Durch diese Lösung wurde zur Aktivierung des Katalysators eine Stunde lang Kohlenmonoxid (25 l/h) geleitet. Anschließend wurden 1,078 g Mangan(III)acetylacetonat und 2,12 g Natriumphenolat gelöst in 50 g Phenol zugegeben, unter Einleitung eines Gasgemisch aus Kohlenmonoxid und Sauerstoff (96,5:3,5 Vol.-%) der Druck auf 10 bar eingestellt und die Reaktortemperatur auf 80°C erhöht. Die Menge an Gasgemisch, bestehend aus Kohlenmonoxid und Sauerstoff, wurde auf 280 Nl/h eingestellt. Dem Reaktionsgemisch wurde jede Stunde eine Probe entnommen und gaschromatographisch analysiert. Die Analysen ergaben, dass nach einer Stunde 30,1 Gew.-% Diphenylcarbonat, nach 2 Stunden 38,5 Gew.-% Diphenylcarbonat und nach 3 Stunden 46,0 Gew.-% Diphenylcarbonat im Reaktionsgemisch enthalten waren. In den Kühlfallen waren 16,7 g eines Phenol/Wasser-Gemisches kondensiert.

Die Phenol-Selektivität lag mit > 99 % unverändert hoch. Nebenprodukte aus einer DPC-Abreaktion oder größere CO₂-Mengen aufgrund einer DPC-Hydrolyse wurden nicht beobachtet.

### Beispiel 2: (35,7 % DPC-Zusatz)

In einem 1 Autoklaven mit Begasungsrührer, Wellenbrecher, kontinuierlicher Gasdosierung, Druckhaltung, Kühler und nachgeschalteten Kühlfallen, wurden 0,12 g Palladiumbromid und 11,64 g Tetrabutylammoniumbromid bei 60°C in 250 g DPC und 400 g Phenol gelöst. Durch diese Lösung wurde zur Aktivierung des Katalysators eine Stunde lang Kohlenmonoxid (25 l/h) geleitet. Anschließend wurden 1,078 g Mangan(III)acetylacetonat und 2,71 g Kaliumphenolat gelöst in 50 g Phenol zugegeben, unter Einleitung eines Gasgemisch aus Kohlenmonoxid und Sauerstoff (96,5:3,5 Vol.-%) der Druck auf 9 bar eingestellt und die Reaktortemperatur auf 85°C erhöht. Die Menge an Gasgemisch, bestehend aus Kohlenmonoxid und Sauerstoff, wurde auf 320 Nl/h eingestellt. Dem Reaktionsgemisch wurde jede Stunde eine Probe entnommen und gaschromatographisch analysiert. Die Analysen ergaben, dass nach einer Stunde 43,9 Gew.-% Diphenylcarbonat, nach 2 Stunden 51,9 Gew.-% Diphenylcarbonat und nach 3 Stunden 59,8 Gew.-% Diphenylcarbonat im Reaktionsgemisch enthalten waren. In den Kühlfallen waren 17,8 g eines Phenol/Wasser-Gemisches kondensiert.

Die Phenol-Selektivität lag mit > 99 % unverändert hoch. Nebenprodukte aus einer DPC-Abreaktion oder größere CO₂-Mengen aufgrund einer DPC-Hydrolyse wurden nicht beobachtet.

### Beispiel 3: (50 % DPC-Zusatz)

In einem Autoklaven (1l) mit Begasungsrührer, Wellenbrecher, kontinuierlicher Gasdosierung, Druckhaltung, Kühler und nachgeschalteten Kühlfallen, wurden 0,16 g Palladiumbromid und 11,64 g Tetrabutylammoniumbromid bei 60°C in 350 g DPC und 300 g Phenol gelöst. Durch diese Lösung wurde zur Aktivierung des Katalysators eine Stunde lang Kohlenmonoxid (25 l/h) geleitet. Anschließend wurden 1,078 g Mangan(III)acetylacetonat und 2,115 g Natriumphenolat gelöst in 50 g Phenol zugegeben, unter Einleitung eines Gasgemisch aus Kohlenmonoxid und Sauerstoff (96,5:3,5 Vol.-%) der Druck auf 12 bar eingestellt und die Reaktortemperatur auf 90°C. Die Menge an Gasgemisch, bestehend aus Kohlenmonoxid und Sauerstoff, wurde auf 400 Nl/h eingestellt. Dem Reaktionsgemisch wurde jede Stunde eine Probe entnommen und gaschromatographisch analysiert. Die Analysen ergaben, dass nach einer Stunde 58,1 Gew.-% Diphenylcarbonat, nach 2 Stunden 66,0 Gew.-% Diphenylcarbonat und nach 3 Stunden 73,8 Gew.-% Diphenylcarbonat im Reaktionsgemisch enthalten waren. In den Kühlfallen kondensierten 10,4 g eines Phenol/Wasser-Gemisches.

### Beispiel 4:

### Belegung eines pulverförmigen Titandioxids mit Palladium und Mangan:

Zu einer Aufschlämmung von 283,5 g Titandioxid-Pulver (Hersteller: Norton) in 1500 ml Wasser wurden bei Raumtemperatur 300 ml Lösung von 40,5 g (0,16 mol) Mangan(II)-nitrat-4-hydrat in Wasser gegeben. Dann wurde mit verdünnter Natronlauge alkalisch gestellt. Die Suspension wurde abgesaugt, mit Wasser gewaschen, bei 100°C getrocknet und 3 h bei 300°C getempert. Der mit Mangan dotierte Träger wurde in 1500 ml Wasser aufgeschlämmt und mit 300 ml Lösung, enthaltend 50 g Natriumtetrachloropalladat(II)-Lsg. mit 15 Gew.-% Palladium, versetzt. Dann wurde mit verdünnter Natronlauge alkalisch gestellt. Die Suspension wurde abgesaugt, gewaschen und bei 100°C getrocknet.

Der Katalysator enthält 2,5 Gew.-% Pd und 3 Gew.-% Mn, jeweils als Metall gerechnet.

### Einsatz des Trägerkatalysators zur Herstellung von Diphenylcarbonat:

Das Beispiel 1 wurde wiederholt, wobei statt 0,12 g Palladiumbromid 2 g des oben hergestellten heterogenen Katalysators in Suspension eingesetzt und bei 8 bar gearbeitet wurde.

Die Analysen ergaben, dass nach einer Stunde 28,3 Gew.-% Diphenylcarbonat, nach 2 Stunden 35,1 Gew.-% Diphenylcarbonat und nach 3 Stunden 41,8 Gew.-% Diphenylcarbonat im Reaktionsgemisch enthalten waren. In den Kühlfallen 21,3 g eines Phenol/Wasser-Gemisches kondensiert.

### Beispiel 5:

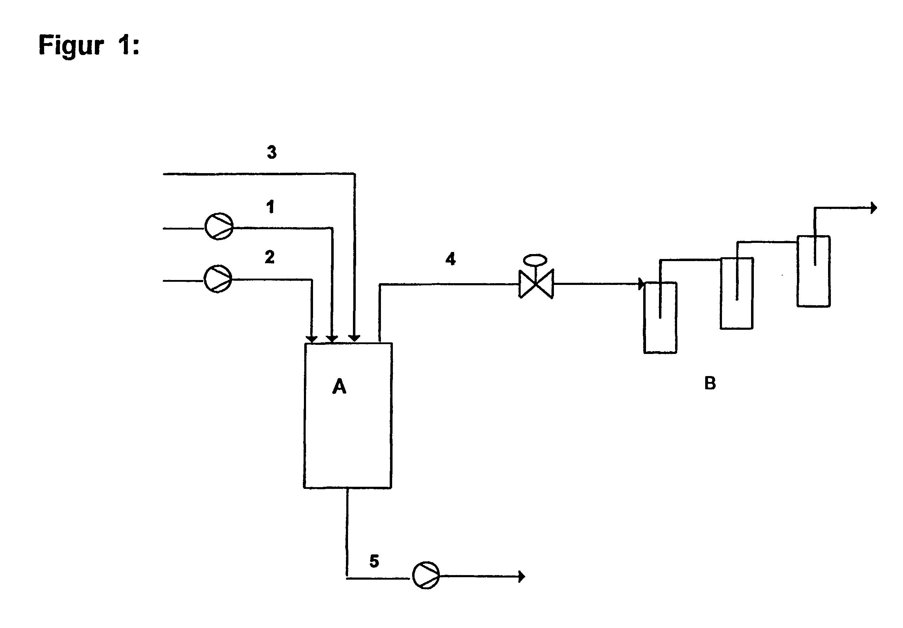

Mit der in Figur 1 schematisch dargestellten Apparatur, bestehend aus einem 11 Autoklaven (A), kontinuierlicher Gas- und Eduktdosierung, kontinuierlicher Gasund Flüssigkeitsausschleusung wurde ein kontinuierlicher Versuch durchgeführt. Das Reaktionsgas durchströmte nach der Entspannung drei Kühlfallen (B), wobei ausgestripptes Wasser und Phenol kondensierten.

Über Leitung 1 förderte eine Pumpe die aktivierte Katalysatorlösung (pro Stunde 0,10 g Palladiumbromid, 8,31 g Tetrabutylammoniumbromid und 1,07 g Mangan-(III)-acetylacetonat in einer Schmelze aus 100 g DPC und 350 g Phenol) in den Reaktor. Gleichzeitig dosierte eine weitere Pumpe über Leitung 2 pro Stunde 1,511 g Natriumphenolat gelöst in 50 g Phenol in den Reaktor.

Die Temperatur der Reaktionslösungen betrug 80°C. Über Leitung 3 gelangten pro Stunde 300 N1 Gasgemisch, bestehend aus Kohlenmonoxid und Sauerstoff (96,5:3,5 Vol.-%) in den Reaktor. Der Reaktordruck betrug 10 bar und die Innentemperatur wurde auf 80°C geregelt. Überschüssiges Reaktionsgas verließ über Leitung 4 den Reaktor. Der Reaktorinnendruck wurde über einen Druckaufnehmer und ein Regelventil in Leitung 4 konstant gehalten.

Mit Hilfe einer Pumpe wurden über Leitung 5 pro Stunde ca. 500 g Reaktionsgemisch aus dem Reaktor entnommen und der Diphenylcarbonatisolierung zugeführt. Dem ausgeschleusten Reaktionsgemisch wurde stündlich eine Probe entnommen und gaschromatographisch analysiert. Nach ca. 5 Stunden war die Apparatur im Gleichgewicht. Die Analysen ergaben, dass in den Reaktionsgemischen 28,2 Gew.-% Diphenylcarbonat enthalten waren. Die Phenol-Selektivität lag mit > 99 % unverändert hoch.

Nebenprodukte aus einer DPC-Abreaktion oder größere CO₂-Mengen infolge einer DPC-Hydrolyse wurden nicht beobachtet.

### Beispiel 6:

Es wurde wie nach Beispiel 5 verfahren, mit dem Unterschied, dass pro Stunde 0,10 g Palladiumbromid, 8,31 g Tetrabutylammoniumbromid und 1,07 g Mangan-(III)-acetylacetonat in einem Gemisch aus 250 g DPC und 200 g Phenol in den Reaktor gefördert wurden.

Dem ausgeschleusten Reaktionsgemisch wurde stündlich eine Probe entnommen und gaschromatographisch analysiert. Nach ca. 5 Stunden war die Apparatur im Gleichgewicht. Die Analysen ergaben, dass in den Reaktionsgemischen 58,7 Gew.-% Diphenylcarbonat enthalten waren. Die Phenol-Selektivität lag mit >99 % unverändert hoch.

Nebenprodukte aus einer DPC-Abreaktion oder größere CO₂-Mengen infolge einer DPC-Hydrolyse wurden nicht beobachtet

Vor Eintritt in die DPC-Isolierung wurden durch nachträgliche Zugabe von 225 g DPC der Gehalt in der Reaktionsmischung auf ca. 71 Gew.-% DPC gesteigert.

Die DPC-Isolierung erfolgte nach EP-A 687 666 in einem Röhrenkristaller durch fraktionierte Schmelzkristallisation.

Die Reaktionsmischung wurde in ein senkrecht stehendes ummanteltes Rohr von 100 cm Höhe und ca. 3 cm Innendurchmesser gegeben und von 75°C mit 2°C/h abgekühlt. Bei 68°C wurde die Schmelze mit einigen Kristallen Diphenylcarbonat angeimpft. Als die Schmelze 55°C erreichte, wurde sie abgelassen. Anschließend wurde das Heizmedium mit 2°C wieder aufgeheizt. Nach Erreichen von 69°C wurde die im Rohr verbliebene Kristallmasse abgeschmolzen und separat aufgefangen. Sie enthielt 92 Gew.-% DPC. Die insgesamt zuvor abgetrennte Schmelze von ca. 437 g bestand zu 57,2 Gew.-% aus Diphenylcarbonat. Das Kristallisat wurde anschließend destillativ von restlichem Phenol befreit.

## Patentansprüche

1. Verfahren zur Herstellung eines organischen Carbonats der Formel
R-O-CO-O-R (I)
in der
R ein substituiertes oder nicht substituiertes C₆-C₁₂-Aryl, bevorzugt ein substituiertes oder nicht substituiertes Phenyl, besonders bevorzugt nicht substituiertes Phenyl bedeutet,
durch Umsetzung einer aromatischen Hydroxyverbindung der Formel
R-O-H (II)
worin
R die oben angegebene Bedeutung hat,
mit Kohlenmonoxid und Sauerstoff in Gegenwart eines Platinmetall-Katalysators, eines Cokatalysators, eines quaternären Salzes und einer Base bei einer Temperatur von 30 bis 200°C und einem Druck von 1 bis 200 bar, das **dadurch gekennzeichnet ist, dass** man die Reaktion in einer Schmelze aus Diarylcarbonat und aromatischer Hydroxyverbindung durchführt, die schon zu Beginn der Reaktion einen Gehalt an Diarylcarbonat von mindestens 20 Gew.-% aufweist und das Reaktionswasser durch Strippen mit Reaktionsgas aus der Reaktionsmischung entfernt und aus der Schmelze in im Prinzip bekannter Weise Diarylcarbonat isoliert.

2. Verfahren gemäß Anspruch 1, bei dem der Schmelze vor der Isolierung des Diarylcarbonats noch zusätzlich Diarylcarbonat zugesetzt wird.

3. Verfahren gemäß Anspruch 1, bei dem man kontinuierlich einen Teil der Reaktionsmischung entnimmt, aus diesem in im Prinzip bekannter Weise Diarylcarbonat isoliert und diesen anschließend wieder in den Prozess zurückführt.

4. Verfahren gemäß Anspruch 3, bei dem man den entnommenen Teil der Reaktionsmischung vor der Isolierung des Diarylcarbonats noch zusätzlich mit Diarylcarbonat versetzt.

## Claims

1. A process for producing an organic carbonate of formula
R-O-CO-O-R (I)
where
R represents a substituted or unsubstituted C₆-C₁₂ aryl, preferably a substituted or unsubstituted phenyl, most preferably unsubstituted phenyl,
by the reaction of an aromatic hydroxy compound of formula
R-O-H (II)
where
R has the meaning given above,
with carbon monoxide and oxygen in the presence of a platinum metal catalyst, a co-catalyst, a quaternary salt and a base at a temperature of 30 to 200°C and at a pressure of 1 to 200 bar, which is **characterised in that** the reaction is conducted in a melt comprising a diaryl carbonate and an aromatic hydroxy compound, which at the start of the reaction already has a content of diaryl carbonate of at least 20 % by weight, and diaryl carbonate is isolated from the melt in a manner which is known in principle.

2. A process according to claim 1, wherein additional diaryl carbonate is added to the melt before the isolation of the diaryl carbonate.

3. A process according to claim 1, wherein part of the reaction mixture is continuously removed, diaryl carbonate is isolated from this part in a manner which is known in principle, and this part is subsequently recycled to the process.

4. A process according to claim 3, wherein the part of the reaction mixture which is removed is treated in addition with diaryl carbonate before the isolation of the diaryl carbonate.

## Revendications

1. Procédé de fabrication d'un carbonate organique de la formule
R-O-CO-O-R (I)
dans laquelle
R signifie un aryle C₆-C₁₂, substitué ou non substitué, de préférence un phényle substitué ou non substitué, de manière particulièrement préférée un phényle non substitué,
par réaction d'un composé hydroxylé aromatique de la formule
R-O-H (II)
dans laquelle
R a la signification indiquée ci-dessus,
avec du monoxyde de carbone et de l'oxygène en présence d'un catalyseur à base d'un métal du groupe du platine, d'un co-catalyseur, d'un sel quaternaire et d'une base à une température de 30 à 200°C et une pression de 1 à 200 bars, **caractérisé en ce qu'**on réalise la réaction dans une matière fondue de carbonate de diaryle et d'un composé hydroxylé aromatique qui présente déjà au début de la réaction une teneur en carbonate de diaryle d'au moins 20 % en poids et qu'on extrait l'eau de réaction du mélange de réaction par stripage avec le gaz de réaction et qu'on isole le carbonate de diaryle de la matière fondue selon un procédé en principe connu.

2. Procédé selon la revendication 1, dans lequel on ajoute encore du carbonate de diaryle à la matière fondue avant la séparation du carbonate de diaryle.

3. Procédé selon la revendication 1, dans lequel on prélève en continu une partie du mélange de réaction, dont on isole le carbonate de diaryle selon un procédé en principe connu et qu'on recycle ensuite celui-ci dans le processus.

4. Procédé selon la revendication 3, dans lequel on ajoute encore du carbonate de diaryle à la partie prélevée du mélange de réaction avant la séparation du carbonate de diaryle.
